Europäisches Patentamt

European Patent Office

Office européen des brevets

(11) Publication number: **0 483 111 A2**

## EUROPEAN PATENT APPLICATION

(21) Application number: **92101227.4**

(22) Date of filing: **07.10.88**

(51) Int. Cl.⁵: **A61K 7/16**

This application was filed on 27 - 01 - 1992 as a divisional application to the application mentioned under INID code 60.

(30) Priority: **08.10.87 US 106733**

(43) Date of publication of application:
**29.04.92 Bulletin 92/18**

(60) Publication number of the earlier application in accordance with Art.76 EPC: **0 311 412**

(84) Designated Contracting States:
**AT BE CH DE FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Glandorf, William Michael**
**48 Dusk Court**
**Fairfield, Ohio 45014(US)**
Inventor: **Seus, John David**
**3762 Ault Park Avenue**
**Cincinnati, Ohio 45208(US)**
Inventor: **White, Donald James, Jr.**
**5690 Bordeaux Way**
**Fairfield, Ohio 45014(US)**

(74) Representative: **Brooks, Maxim Courtney et al**
**Procter & Gamble (NTC) Limited Whitley Road Longbenton**
**Newcastle-upon-Tyne NE12 9TS(GB)**

(54) **Anticalculus compositions.**

㊐ Disclosed are oral compositions, toothpastes, mouthrinses and liquid dentifrices, containing potassium and sodium pyrophosphates in amounts to achieve desired concentrations of pyrophosphate ions while maintaining good stability and minimizing other problems.

EP 0 483 111 A2

TECHNICAL FIELD

The present invention relates to oral compositions containing pyrophosphate salts which provide an anticalculus benefit and are stabilized against pyrophosphate crystal growth.

BACKGROUND OF THE INVENTION

Dental calculus, or tartar as it is sometimes called, is a deposit which forms on the surfaces of the teeth at the gingival margin. Supragingival calculus appears principally in the areas near the orifices of the salivary ducts; e.g., on the lingual surfaces of the lower anterior teeth and on the buccal surfaces of the upper first and second molars, and on the distal surfaces of the posterior molars.

Mature calculus consists of an inorganic portion which is largely calcium phosphate arranged in a hydroxyapatite crystal lattice structure similar to bone, enamel and dentine. An organic portion is also present and consists of desquamated epithelial cells, leukocytes, salivary sediment, food debris and various types of microorganisms.

As the mature calculus develops, it becomes visibly white or yellowish in color unless stained or discolored by some extraneous agent. This is undesirable from an aesthetic standpoint.

A wide variety of chemical and biological agents have been suggested in the art to retard calculus formation or to remove calculus after it is formed. Mechanical removal of this material periodically by the dentist is, of course, routine dental office procedure.

The chemical approach to calculus inhibition generally involves chelation of calcium ion and/or crystal growth inhibition which prevents the calculus from forming and/or breaks down mature calculus by removing calcium.

The prior art discloses a number of chelating agents for this purpose. British Patent 490,384, February 15, 1937, discloses oral compositions containing ethylenediaminetetraacetic acid, nitrilotriacetic acid and related compounds as anticalculus agents. U.S. Patent 3,678,154, July 18, 1972 to Widder et al. discloses oral compositions containing certain polyphosphonates and fluoride. U.S. Patent 3,737,533, June 5, 1973 to Francis discloses oral compositions containing certain carbonyl diphosphonates.

In addition to the above references, the prior art discloses dentifrices and mouthwashes containing soluble pyrophosphate salts which have been included for a variety of purposes. Included among such references are U.S. Patent 2,941,926, June 21, 1960 to Salzmann et a. which discloses dental powders containing chlorophyll and pyrophosphate salts. U.S. Patent 3,137,632, June 16, 1964 to Schiraldi discloses toothpastes containing pyrophosphate salts. U.S. Patents 3,927,201 and 202, December 16, 1975 to Baines et al. and Harvey et al., respectively, disclose toothpastes which utilize soluble pyrophosphates as abrasives. U.S. Patents 4,244,931, January 13, 1981 and 4,247,526, January 27, 1981 to Jarvis et al. disclose pyrophosphate salts in dicalcium phosphate systems. Jap. Patent Application Disclosure No 4945-1974 discloses soluble pyrophosphates in a variety of dentifrice systems. U.S. Patent 4,333,551, April 6, 1982 to Parran discloses tetraalkali metal salts in mouthwash compositions. U.S. Patent 4,515,772, May 7, 1985 to Parran, et al., discloses compositions containing soluble pyrophosphate salts as anticalculus agents. Parran et al. achieve soluble pyrophosphate systems through the use of acid pyrophosphate salts. U.S. Patent 4,627,977, December 9, 1986 to Gaffar et al. discloses pyrophosphate salts combined with linear anionic polymers in oral compositions.

In addition to the use of the above mentioned materials the use of certain acrylic acid polymers and other agents have also been disclosed for use as anticalculus agents. Included among such agents are polyelectrolytes such as copolymers of maleic anhydride and ethylene disclosed in U.S. Patent 3,429,963, February 25, 1969 to Shedlovsky. Shedlovsky also discloses polyacrylic acid having an average molecular weight of 1500 and greater. Another reference disclosing polyacrylic acids in oral compositions is South African Patent 720,898, September 12, 1972 which discloses such acids having a molecular weight in the range of 2,000 to 4,000,000 for use as a membrane to prevent the elution from teeth of previously applied agents. U.S. Patent 3,956,480, May 11, 1976 to Gaffar discloses complexes of anionic polymers (e.g. acrylic acid) and a cationic therapeutic agent (e.g., chlorhexidine) as anticalculus agents. Strontium chelates have also been disclosed for use in oral compositions, particularly in the enhancement of fluoride uptake.

The effect of a strontium-EDTA complex in combination with sodium ricinoleate and a fluoride source is found in the Journal of Dental Research (1982) 61 (3) 451-455. The combined effect of strontium and fluoride in reducing the acid solubility of enamel is also disclosed in the Journal of Dental Research (1983) 62 (10) 1049-1053. A further reference discussing the effect of strontium and fluoride is Featherstone, J.D.B. "Remineralization of Artificial Carious Lesions In-vivo and In-vitro", Proceedings Workshop (1983) IRL Press Ltd.

The use of strontium in combination with fluoride in oral compositions is also disclosed in a number of patent references. Included among these references are U.S. Patent 3,888,976, June 10, 1975 to Mlkvys disclosing an effervescent mouthwash tablet containing strontium ions and possibly a fluoride ion source. U.S. Patent 4,367,219, January 4, 1983 to Schole discloses dentifrices containing a combination of strontium EDTA, a ricinoleate salt and a fluoride ion source. U.S. Patent 4,425,549, November 15, 1983 to Shah et al. discloses toothpastes containing a glycyrrhizinate salt, strontium EDTA and a fluoride ion source. Finally European Patent Application 0,079,611, June 6, 1983, Shah, discloses oral compositions containing a strontium EDTA complex and a fluoride ion source.

Although there have been a number of approaches disclosed for combatting calculus, there is still the desire and need to develop improved products possessing that property. The prior art while disclosing the use of pyrophosphate salts provides no suggestion to use such salts in a mixture wherein the salts are present in amounts sufficient to achieve desired pyrophosphate ion levels while avoiding crystal growth and while not relying solely on acid pyrophosphate salts or potassium pyrophosphate salts for that purpose.

It is an object of the present invention to provide compositions which deliver an anticalculus benefit.

It is a further object of the present invention to produce an effective anticalculus product using a mixture of pyrophosphate salts, which impart increased stability and which do not rely on high levels of sodium acid or potassium pyrophosphate salts for avoiding pyrophosphate crystal growth in the product.

It is still a further object of the present invention to provide anticalculus products which are cosmetically acceptable and do not inhibit remineralization of the teeth.

It is still a further object of the present invention to provide effective methods for combating calculus.

These and other objects will become more clear from the detailed description which follows.

All percentages and ratios used herein are by weight unless otherwise specified. Also all measurements referred to herein are made at $25°C$ in the composition unless otherwise specified.

## SUMMARY OF THE INVENTION

The present invention embraces an oral composition comprising:
(a) a safe and effective amount of a mixture of pyrophosphate salts;
(b) a safe and effective amount of a soluble fluoride ion source; and
(c) a pharmaceutically acceptable carrier. wherein the ratio of the ion product of tetrasodium pyrophosphate species to the solubility constant for that material falls within specified ranges for $20°F$ and $80°F$.

The present invention also encompasses a method for retarding development of dental calculus using the compositions described herein.

## DETAILED DESCRIPTION OF THE INVENTION

The compositions of the present invention comprise a mixture of pyrophosphate salts in a pharmaceutically acceptable carrier.

By "oral compositions" as used herein means a product which in the ordinary course of usage is not intentionally swallowed for purposes of systemic administration of particular therapeutic agents, but is rather retained in the oral cavity for a time sufficient to contact substantially all of the dental surfaces and/or oral tissues for purposes of oral activity.

By "safe and effective amount" as used herein means sufficient amount of material to provide the desired benefit while being safe to the hard and soft tissues of the oral cavity.

By the term "comprising", as used herein, is meant that various additional components can be conjointly employed in the compositions of this invention as long as the listed materials perform their intended functions.

By the term "pharmaceutically acceptable carrier", as used herein, is meant a suitable toothpaste, mouthrinse or liquid dentifrice vehicle which can be used to apply the present anticalculus agents in the oral cavity.

### Pyrophosphate Salts

The pyrophosphate salts used in the present compositions can be any of the alkali metal pyrophosphate salts. Specific salts include tetra alkali metal pyrophosphate, dialkali metal diacid pyrophosphate, trialkali metal monoacid pyrophosphate and mixtures thereof, wherein the alkali metals are preferably sodium or potassium. The salts are useful in both their hydrated and unhydrated forms. The amount of pyrophosphate salt useful in the present composition is any effective amount and is generally enough to provide at least 1.0% $P_2O_7^{-4}$, preferably from about 1.5% to about 6%, more preferably from about 3.5% to about 6%, to the compositions. It is to be appreciated that the level of $P_2O_7^{-4}$ is that capable of being provided to the composition (i.e., the theoretical amount at an appropriate pH) and that other pyrophosphate forms (e.g., $HP_2O_7^{-3}$) may be present when a final product pH is established.

Tetrapotassium pyrophosphate is a very soluble pyrophosphate species but suffers from negative flavor aspects. The compositions of the present invention contain this tetra salt but the amount is minimized while still allowing for stable products (i.e., the tendency to form tetrasodium pyrophosphate crystals is reduced).

The pyrophosphate salts are described in more detail in Kirk & Othmer, Encyclopedia of Chemical Technology, Second Edition, Volume 15, Interscience Publishers (1968), incorporated herein by reference.

Tetrapotassium pyrophosphate and other pyrophosphate salts are present, along with other sources of sodium ions, in amounts such that the supersaturation for tetrasodium pyrophosphate, S, falls within certain ranges for temperatures of 20°F and 80°F. Supersaturation is defined as

$$S = \frac{IP}{K_{SP}}$$

for any conditions of pH and temperature. IP, the ion product for $Na_4P_2O_7$, is expressed as $IP = [Na_t]^4 - [P_2O_{7t}]$ where $Na_t$ is the total sodium concentration in the formula and $P_2O_{7t}$ is the total pyrophosphate concentration (soluble) in the formula. $K_{SP}$ is the conditional solubility product for $Na_4P_2O_7$ which varies as a function of both temperature and pH. Because conditional stability constants are used as a function of both temperature and pH, the $P_2O_{7(t)}$ is established without specific analysis of speciation (i.e., $HP_2O_7^{3-}$, $H_2P_2O_7^{2-}$ etc.). Thus, $K_{SP}$ at constant temperature and pH is given by solubility values of $Na_t$ and $P_2O_{7t}$.

The relation of $K_{SP}$ for various pH's has been found to follow these relationships at two temperatures:

at 25°C ln $K_{SP}$ = 36.0 - 17.5 ln [pH]

at 4°C ln $K_{SP}$ = 16.2 - 7.8 ln [pH]

with any pH known, and the ln $K_{SP}$'s calculated at 4°C and 25°C, the $K_{SP}$ at any other temperature can be calculated by:

$\Delta G = RT \ln K_{SP}$ and plotting $\ln K_{SP}$ vs $1/T$ wherein G is the heat of solubilization, R is a constant for the relationship and T is the temperature in °C.

The concentrations of the various elements are corrected for the water content in the compositions to establish the concentrations used in the equations (i.e., if a dentifrice contains 40% water, the weight % of an element in the total composition is multiplied by 2.5 to establish a concentration based on volume in the liquid phase, g/ml).

Water Soluble Fluoride Ion Source

Water-soluble fluoride compounds in the present compositions are present in an amount sufficient to give a fluoride concentration of from about 0.0025% to about 5.0% by weight, preferably from about 0.005% to about 2.0% by weight, to provide pyrophosphate ion stability. Preferred fluorides are sodium fluoride, stannous fluoride, indium fluoride, and sodium monofluorophosphate. Norris et al., U.S. Patent 2,946,735, issued July 26, 1960 and Widder et al., U.S. Patent 3,678,154, issued July 18, 1972 disclose such salts as well as others. Both patents are incorporated herein by reference.

Pharmaceutically Acceptable Carrier

The carrier for the active ingredients herein can be any toothpaste, mouthrinse or liquid dentifrice vehicle suitable for use in the oral cavity. Toothpaste and mouthrinses are the preferred systems.

The abrasive polishing material contemplated for use in the toothpaste compositions of the present invention can be any material which does not excessively abrade dentin. These include, for example, silicas including gels and precipitates, calcium carbonate, dicalcium orthophosphate dihydrate, calcium pyrophosphate, tricalcium phosphate, calcium polymetaphosphate, insoluble sodium polymetaphosphate, hydrated alumina, and resinous abrasive materials such as particulate condensation products of urea and formaldehyde, and others such as disclosed by Cooley et al. in U.S. Patent 3,070,510, December 25, 1962, incorporated herein by reference. Mixtures of abrasives may also be used.

Silica dental abrasives, of various types, can provide the unique benefits of exceptional dental cleaning and polishing performance without unduly abrading tooth enamel or dentin. Silica abrasive materials are also exceptionally compatible with sources of soluble fluoride. For these reasons they are preferred for use herein.

The silica abrasive polishing materials useful herein, as well as the other abrasives, generally have an average particle size ranging between about 0.1 and 30 microns, preferably 5 and 15 microns. The silica abrasive can be precipitated silica or silica gels such as the silica xerogels described in Pader et al., U.S. Patent 3,538,230, issued March 2, 1970 and DiGiulio, U.S. Patent 3,862,307, June 21, 1975, both incorporated herein by reference. Preferred are the silica xerogels marketed under the tradename "Syloid" by W. R. Grace & Company, Davison Chemical Division. Preferred precipitated silica materials include those marketed by the J. M. Huber Corporation under the tradename, "Zeodent", particularly the silica carrying the designation "Zeodent 119". These silica abrasives are described in U.S. Patent 4,340,583, July 29, 1982, incorporated herein by reference.

The abrasive in the toothpaste compositions described herein is present at a level of from about 6% to 70%, preferably from about 15% to about 25%.

Flavoring agents can also be added to toothpaste compositions. Suitable flavoring agents include oil of wintergreen, oil of peppermint, oil of spearmint, oil of sassafras, and oil of clove. Sweetening agents which can be used include aspartame, acesulfame, saccharin, dextrose, levulose and sodium cyclamate. Flavoring and sweetening agents are generally used in toothpastes at levels of from about 0.005% to about 2% by weight.

Toothpaste compositions can also contain emulsifying agents. Suitable emulsifying agents are those which are reasonably stable and foam throughout a wide pH range, including non-soap anionic, nonionic, cationic, zwitterionic and amphoteric organic synthetic detergents. Many of these suitable agents are disclosed by Gieske et al. in U.S. Patent 4,051,234, September 27, 1977, incorporated herein by reference. The emulsifying agents are present at a level of from about 0.5% to about 2.0%.

Water is also present in the toothpastes of this invention. Water employed in the preparation of commercially suitable toothpastes should preferably be deionized and free of organic impurities. Water generally comprises from about 10% to 50%, preferably from about 30% to 50%, by weight of the toothpaste compositions herein. These amounts of water include the free water which is added plus that which is introduced with other materials such as with sorbitol.

In preparing toothpastes, it is necessary to add some thickening material to provide a desirable consistency. Preferred thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture. Thickening agents in an amount from 0.2% to 5.0% by weight of the total composition can be used.

It is also desirable to include some humectant material in a toothpaste to keep it from hardening. Suitable humectants include glycerin, sorbitol, and other edible polyhydric alcohols at a level of from about 15% to about 70%.

Another preferred embodiment of the present invention is a mouthwash composition. Conventional mouthwash composition components can comprise the carrier for the agents of the present invention. Mouthwashes generally comprise from about 20:1 to about 2:1 of a water/ethyl alcohol solution and preferably other ingredients such as flavor, sweeteners, humectants and sudsing agents such as those mentioned above for dentifrices. The humectants, such as glycerin and sorbitol give a moist feel to the mouth. Generally, on a weight basis the mouthwashes of the invention comprise 5% to 60% (preferably 5% to 20%) ethyl alcohol, 0% to 20% (preferably 5% to 20%) of a humectant, 0% to 2% (preferably 0.01% to 1.0%) emulsifying agents, 0% to 0.5% (preferably 0.005% to 0.06%) sweetening agent such as saccharin, 0% to 0,3% (preferably 0.03% to 0.3%) flavoring agent, and the balance water. Other optional components described herein earlier for use in toothpaste products are also useful in the mouthwash compositions.

The pH of the present compositions and/or the pH in the mouth can be any pH which is safe for the mouth's hard and soft tissues. Such pH's are generally from about 3 to about 10, preferably from about 4 to about 8.

A method of manufacture for the present compositions is found in the examples.

COMPOSITION USE

The present compositions are used in a conventional manner wherein the amounts of product are what users generally use.

The following examples further describe and demonstrate preferred embodiments within the scope of the present invention. The examples are given solely for illustration and are not to be construed as limitations of this invention as many variations thereof are possible without departing from the spirit and scope thereof.

EXAMPLE I

The following is a composition representative of the present invention.

| Component | Wt. % |
|---|---|
| Sorbitol (70% Aqueous Solution) | 23.000 |
| Sodium Fluoride | 0.243 |
| Sodium Saccharin | 0.345 |
| Tetrapotassium Pyrophosphate (65% Aqueous Solution) | 5.881 |
| Tetrasodium Pyrophosphate | 2.050 |
| Sodium Acid Pyrophosphate | 2.100 |
| PEG-6 | 3.000 |
| Dye | 0.050 |
| Flavor | 1.000 |
| Titanium Dioxide | 0.500 |
| Silica Abrasive | 22.000 |

| | |
|---|---|
| Glycerin | 9.000 |
| Sodium Alkyl Sulfate (27% Aqueous Solution) | 4.000 |
| Xanthan Gum | 0.600 |
| Carbopol-941[1] | 0.200 |
| Water | 26.031 |
| | 100.000% |

pH in neat product = 6.8

[1] A carboxyvinyl polymer sold by B. F. Goodrich Company

The ion product, IP, for this product is 5.66.

The conditional $K_{SP}$'s for $Na_4P_2O_7$ at 20°F and at 80°F at a formula pH of 6.8 are 1.84 and 12.99 respectively.

Therefore the supersaturation,

$$S = \frac{IP}{K_{SP}},$$

equals 2.57 at 20°F and 0.36 at 80°F.

EXAMPLE II

The following is another composition representative of the present invention.

| Component | Wt. % |
|---|---|
| Sorbitol (70% Aqueous Solution) | 32.000 |
| Sodium Fluoride | 0.243 |
| Sodium Saccharin | 0.322 |
| Tetrapotassium Pyrophosphate (65% Aqueous Solution) | 5.910 |
| Tetrasodium Pyrophosphate | 2.100 |
| Sodium Acid Pyrophosphate | 0.750 |
| PEG-6 | 4.000 |
| Dye | 0.050 |
| Flavor | 0.950 |
| Titanium Dioxide | 0.525 |
| Silica Abrasive | 21.000 |
| Glycerin | 8.000 |
| Sodium Alkyl Sulfate | 4.000 |
| Xanthan Gum | 0.500 |
| Carbopol-941 | 0.250 |
| Water | 19.400 |
| | 100.000 |
| Neat pH 7.3 | |
| The supersaturation of this product at 20°F is 1.78 while at 80°F it is 0.71. | |

Claims

1. An anticalculus oral composition in the form of a toothpaste, mouthrinse or liquid dentifrice comprising:

a) an amount of a mixture of water soluble alkali metal pyrophosphate ion sources, including some tetrapotassium pyrophosphate, sufficient to provide at least 1.0% by weight $P_2O_7^{-4}$;

b) a safe and effective amount of a water soluble fluoride ion source;

c) a pharmaceutically acceptable carrier containing water; wherein said composition contains sodium ions such that the supersaturation value, S, for $Na_4 P_2O_7$ in said composition is less than 3.4 at 20°F and between 0.3 and 1.0 at 80°F fora given formula pH, and wherein are excluded oral compositions comprising a mixture of water-soluble alkali metal pyrophosphate ion sources in an amount sufficient to provide at least 3.5% $P_2O_7^{-4}$.

2. An anticalculus composition according to Claim 1 wherein the pyrophosphate ion source is a mixture of tetrapotassium pyrophosphate and a salt selected from the group consisting of sodium acid pyrophosphate, tetrasodium pyrophosphate and mixtures thereof.

3. An anticalculus composition according to any of Claims 1-2 wherein the fluoride ion source is selected from the group consisting of sodium fluoride, stannous fluoride, and mixtures thereof.

4. An anticalculus composition according to any of Claims 1-3 which is in the form of a toothpaste.

5. An anticalculus composition according to Claim 4 which in addition contains a dental abrasive.

6. An anticalculus composition according to any of Claims 1-5 wherein the fluoride ion source is sodium fluoride.

7. An anticalculus composition according to Claim 5 wherein the dental abrasive is a silica dental abrasive.

8. An anticalculus composition according to any of Claims 1-3 which is in the form of a mouthrinse.

9. An anticalculus composition according to Claim 8 which in addition contains a humectant and ethanol.

10. An anticalculus composition according to Claim 9 wherein the fluoride ion source is sodium fluoride.

11. An oral composition according to Claim 3 in the form of a liquid dentifrice.